# EUROPÄISCHE PATENTSCHRIFT

(11) **EP 0 437 174 B1**
(45) Veröffentlichungstag und Bekanntmachung des Hinweises auf die Patenterteilung: **22.12.1993**
(21) Anmeldenummer: 90810865.7
(22) Anmeldetag: 09.11.1990
(51) Int. Cl.: A61F 2/08

(54) **Implantat für einen künstlichen Bänder- und/oder Sehnenersatz**
Artificial ligament and/or tendon replacement implant
Implant pour substitution artificielle des ligaments et/ou tendons

(30) Priorität: 08.01.1990 CH 46/90
(43) Veröffentlichungstag der Anmeldung: 17.07.1991
(73) Patentinhaber: SULZER Medizinaltechnik AG, 8404 Winterthur (CH); PROTEK AG, 3110 Münsingen-Bern (CH)
(72) Erfinder: Freudiger, Stefan, CH-3047 Bremgarten (DE); Flückiger, Hans, CH-8128 Hinteregg (CH); Koch, Rudolf, CH-8267 Berlingen (CH)
(74) Vertreter: Trieblnig, Adolf

(56) Entgegenhaltungen:
- EP-A- 0 126 520
- WO-A-89/01320
- FR-A- 2 278 313
- US-A- 4 209 859

## Beschreibung

Die Erfindung betrifft ein Implantat für einen künstlichen Bänder- und/oder Sehnenersatz aus einem bandartigen, textilen Flächengebilde, bei dem die in Längsrichtung verlaufenden Fadenstränge aus einer Anzahl einzelner Fäden bestehen, die durch Zwirnen, Flechten oder Seilen in ihrer Festigkeit verstärkt sind, und bei dem ferner die Fadenstränge mindestens in Abschnitten ihrer Länge durch sie umschlingende Querfäden verfestigt sind, wobei zwischen zwei um die Längsachse gefalteten Teilbereichen der durch Querfäden verstärkten Implantatabschnitte ein Abschnitt vorhanden ist, in dem das Implantat nur aus den in Längsrichtung verlaufenden Fadensträngen besteht und wobei die Falten in den Teilbereichen durch Verbinden der seitlichen Kanten mindestens an einzelnen Stellen fixiert sind.

Natürliche Bänder, die ja aus einzelnen Fasern in Längsrichtung aufgebaut sind, sind bekanntlich aus sich kreuzenden Faserbündeln zusammengesetzte, dreidimensionale "Gebilde", die bei einer Belastung ausschliesslich auf Zug beansprucht werden, ohne dass einzelne von ihnen gestaucht werden. Dabei übernehmen zunächst ein oder einige wenige Faserbündel die "Hauptlast", wobei bei steigenden Lasten und Dehnungen weitere Bündel wirksam werden.

Ein Implantat der eingangs geschilderten Art ist aus der FR-A-22 78 313 bekannt; bei einer der dortigen Konstruktionen schliesst sich beidseitig an einen funktionellen Bereich aus einem textilen Flächengebilde, das in von einer Kunststoff-Folie umhüllt und mit dieser verschweisst- sowie mehrfach gefaltet - ist, je ein Abschnitt an, der nur aus in Längsrichtung verlaufenden Fadensträngen besteht. Diese Abschnitte dienen dazu ein Vernähen des künstlichen Bandes mit einer natürlichen Sehne oder einem natürlichen Band zu ermöglichen.

Weitere Konstruktionen künstlicher Sehnen und/oder Bänder sind beispielsweise in den US-Patenten 4,246,660, 4,187,558 und 3,513,484 gezeigt und beschrieben. Bei allen diesen bekannten Konstruktionen sind die künstlichen Implantate - im Gegensatz zu den natürlichen Bändern - flache, ebene Gebilde, die im wesentlichen zweidimensional ausgerichtet sind. Werden derartige Implantate, z.B. an der Kante der Verankerungsbohrung, auf Biegung beansprucht, so erfahren einzelne Längsfäden eine Zugbelastung, während andere gestaucht werden.

Aufgabe der Erfindung ist es, - ausgehend von den Implantaten nach der FR-A-22 78 313 - einen Bänder- und/oder Sehnenersatz zu schaffen, dessen Wirkungsweise die geschilderte Anatomie und Physiologie natürlicher Bänder möglichst weitgehend nachahmt.

Diese Aufgabe wird mit der Erfindung dadurch gelöst, dass die zwei aufeinanderfolgenden gefalteten Bereiche, welche zwischen sich einen nur aus in Längsrichtung verlaufenden Fadensträngen bestehenden Abschnitt aufweisen, in zueinander entgegengesetzten Richtungen gefaltet sind.

Durch das Falten einzelner Bereiche verformt sich das Implantat zu einem dreidimensionalen Körper, bei dem unter natürlicher Belastung des Bandes die in Längsrichtung verlaufenden Fadenstränge bei genügender Vorspannung nur noch auf Zug belastet - und nicht mehr gestaucht - werden.

Durch das Falten des Bandes werden das Implantieren durch eine Knochenbohrung hindurch un das Verankern am Knochen erleichtert. Dabei kann es vorteilhaft sein, wenn die gefalteten Bereiche in bekannter Weise "gerollt" werden, d.h. wenn sich die seitlichen Kanten dieser Bereiche überlappen.

Das Umschlingen der in Längsrichtung verlaufenden Fadenstränge durch Querfäden kann auf für die Herstellung textiler Flächengebilde oder Bänder bekannte Weise, beispielsweise durch Weben, Flechten, Wirken usw. erfolgen.

Im folgenden wird die Erfindung anhand von Ausführungsbeispielen im Zusammenhang mit der Zeichnung näher erläutert.
- Fig. 1: gibt in einer Ansicht, zwei durch Querfäden verstärkte Abschnitte eines künstlichen Bandes im ungefalteten Zustand wieder, die durch einen nur aus in Längsrichtung verlaufenden Fadensträngen bestehenden Abschnitt getrennt sind;
- Fig. 2: verdeutlicht in gleicher Darstellung wie Fig. 1 die Querelastizität und Flexibilität dieses künstliches Bandes;
- Fig. 3: zeigt ein Band nach Fig. 1 im erfindungsgemäss gefalteten Zustand, während
- Fig. 4: einen Querschnitt durch einen gefalteten Bereich darstellt, bei dem sich die seitlichen Kanten überlappen.

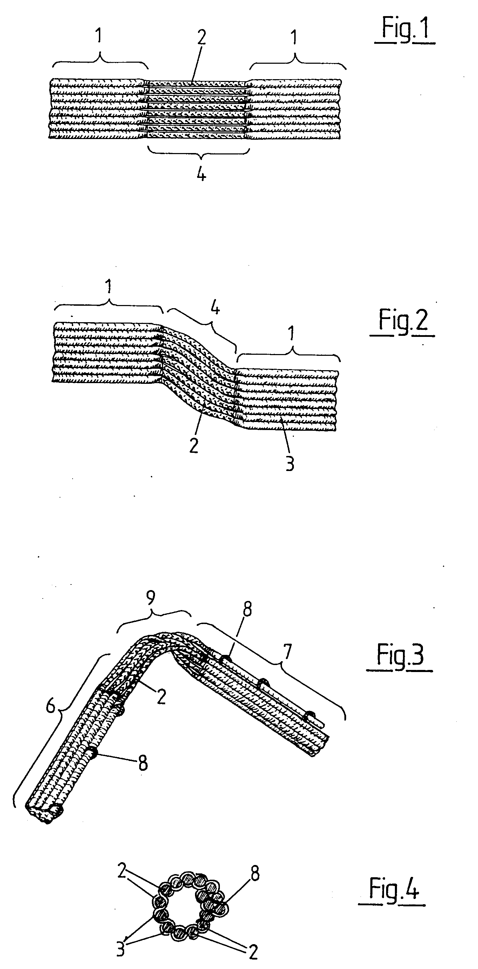

Fig. 1 stellt einen Abschnitt 1 eines Implantats dar, der aus einzelnen Längsfäden 2 besteht, die durch Querfäden 3 zu einem stabilen textilen Band verfestigt sind. Die durch Querfäden 3 verstärkten Abschnitte 1 sind durch einen Abschnitt 4 getrennt, der nur aus in Längsrichtung des Bandes verlaufenden Fadensträngen 2 besteht. Ein solcher Abschnitt 4 verleiht dem Implantat eine hohe Flexibilität, was in Fig. 2 verdeutlicht ist.

Jeder Fadenstrang 2 setzt sich aus einer Anzahl Einzelfäden zusammen, die in dem gezeigten Beispiel durch Flechten zu dem Strang 2 verarbeitet worden sind. Selbstverständlich sind auch andere, aus der Textiltechnik bekannte Verfahren - wie z.B. Zwirnen oder Seilen - anwendbar, durch die eine Anzahl Einzelfäden zu einem Fadenstrang 2 vereinigt werden können.

Die durch Querfäden 3 verstärkten Abschnitte 1 des Implantates sind in dem Ausführungsbeispiel durch Weben hergestellt. Auch für die Herstellung dieser Abschnitte 1 können andere Herstellungsverfahren der Textiltechnik, wie z.B. Flechten oder Wirken, eingesetzt werden.

Bevorzugte Materialien sowohl für die Fäden der Fadenstränge 2 als auch für die Querfäden 3 sind Polyester und Polyäthylen.

Um das Implantat in seiner Funktionsweise an diejenige eines natürlichen Bandes anzunähern, werden im axialen Abstand voneinander angeorndete Bereiche 6 und 7 (Fig. 3) eines mit Querfäden 3 verfestigten Abschnittes 1 gefaltet, wobei die Bereiche links und rechts von Abschnitt 4 in entgegengesetzten Richtungen gefaltet sind. Hierdurch entsteht ein künstliches Band mit einem besonders flexiblen, funktionellen Bereich 9, in dem die einzelnen Fadenstränge 2 relativ zueinander verschiebbar sind und sich daher mindestens teilweise gegenseitig überkreuzen können. Auf diese Weise wird eine optimale Annäherung der Stränge im Abschnitt 4 des künstlichen Bandes an der Verlauf der Fasern eines natürlichen Bandes erreicht.

Die Falten sind dabei durch lokales oder kontinuierliches Verbinden (z.B. Vernähen) der seitlichen Kanten an den Stellen 8 fixiert.

Während die gefalteten Bereiche 6 und 7 sehr häufig in Knochenbohrungen eingezogen sind, liegt der funktionelle Bereich 9 vorzugsweise im intraartikulären Raum des mit dem Band versorgten Gelenkes.

Die Fixierung 8 der seitlichen Kanten der gefalteten Bereiche 6 und 7, die im gezeigten Beispiel durch Vernähen erfolgt, kann auch auf eine andere Weise, z.B. durch Verkleben oder Verschweissen der seitlichen Kanten, hergestellt werden.

Der Querschnitt eines Abschnittes 1 nach Fig. 4 verdeutlicht, dass die Falte auch so ausgeführt sein kann, dass sich die seitlichen Kanten eines Abschnittes 1 im gefalteten Bereich überlappen. Hierdurch erhalten die gefalteten Bereiche eine erhöhte Steifigkeit sowie einen geringeren Durchmesser, wodurch besonders das Ein- oder Durchziehen des künstliches Bandes in oder durch eine Knochenbohrung erleichtert wird.

## Patentansprüche

1. Implantat für einen künstlichen Bänder- und/oder Sehnenersatz aus einem bandartigen, textilen Flächengebilde, bei dem die in Längsrichtung verlaufenden Fadenstränge (2) aus einer Anzahl einzelner Fäden bestehen, die durch Zwirnen, Flechten oder Seilen in ihrer Festigkeit verstärkt sind, und bei dem ferner die Fadenstränge (2) mindestens in Abschnitten ihrer Länge durch sie umschlingende Querfäden (3) verfestigt sind, wobei zwischen zwei um die Längsachse gefalteten Teilbereichen (6, 7) der durch Querfäden (3) verstärkten Implantatabschnitte (1) ein Abschnitt (4) vorhanden ist, in dem das Implantat nur aus den in Längsrichtung verlaufenden Fadensträngen (2) besteht und wobei die Falten in den Teilbereichen (6, 7) durch Verbinden der seitlichen Kanten mindestens an einzelnen Stellen (8) fixiert sind, dadurch gekennzeichnet, dass die zwei aufeinanderfolgenden gefalteten Bereiche (6, 7), welche zwischen sich einen nur aus in Längsrichtung verlaufenden Fadensträngen (2) bestehenden Abschnitt (4) aufweisen, in zueinander entgegengesetzten Richtungen gefaltet sind.

2. Implantat nach Anspruch 1, dadurch gekennzeichnet, dass sich die seitlichen Kanten überlappen.

## Claims

1. An implant for a prosthetic ligament and/or tendon replacement, comprising a flat band-like textile structure in which the longitudinally extending filament strands (2) comprise a number of discrete filaments strengthened by twisting or braiding or cabling and the filament strands (2) are consolidated at least in parts of their length by transverse filaments (3) extending around them, while between two subzones (6, 7) of the implant portions (1) reinforced by transverse filaments (3), such subzones (6, 7) being folded around the longitudinal axis, there is a part (4) in which the implant consists solely of longitudinally extending filament strands (2) and the folds being secured in the subzones (6, 7) by interconnection of the lateral edges at least at discrete places (8), characterised in that the two consecutive folded zones (6, 7), which have between them a part (4) consisting solely of longitudinally extending filament strands (2), are folded in opposite directions.

2. An implant according to claim 1, characterised in that the lateral edges overlap one another.

## Revendications

1. Implant de prothèse de ligaments et/ou de tendons artificiels formés d'un corps plan textile en bande dans laquelle des cordons de fils (2) orientés dans la direction de la longueur se composent de multiples fils individuels dont la résistance est renforcée par retordage, nattage ou tordage en corde et dans laquelle par ailleurs les cordons de fils (2) sont consolidés au moins dans certaines parties de leur longueur par des fils transversaux (3) qui les entourent, une partie (4), dans laquelle l'implant consiste uniquement en cordons (2) orientés dans la direction de la longueur, se trouvant entre deux zones (6, 7) des parties d'implant (1) consolidées par des fils transversaux (3) qui sont repliées autour de l'axe longitudinal et les plis des zones (6, 7) étant fixés au moins en certains emplacements (8) par liaison des bords latéraux, caractérisé en ce que deux zones repliées qui se succèdent (6, 7) et qui comprennent entre elles une partie (4) se composant uniquement de cordons de fils (2) orientés dans la direction de la longueur sont repliées en sens inverses.

2. Implant selon la revendication 1, caractérisé en ce que les bords latéraux se chevauchent.
